# FASCICULE DE BREVET EUROPEEN

(11) **EP 0 830 144 B1**
(45) Date de publication et mention de la délivrance du brevet: **27.02.2002**
(21) Numéro de dépôt: 96914244.7
(22) Date de dépôt: 25.04.1996
(51) Int. Cl.: A61K 47/02

(54) **COMPOSITIONS PULVERULENTES ET SOLUBLES DANS L'EAU ET LEURS APPLICATIONS**
WASSERLÖSLICHE PULVERZUSAMMENSETZUNGEN UND IHRE VERWENDUNGEN
WATER SOLUBLE, POWDER FORM COMPOSITIONS AND APPLICATIONS THEREOF

(30) Priorité: 26.04.1995 FR 9504980
(43) Date de publication de la demande: 25.03.1998
(73) Titulaire: VIRBAC S.A., F-06516 Carros Cedex (FR)
(72) Inventeur: DERRIEU, Guy, F-06800 Cagnes-sur-Mer (FR); POUGNAS, Jean-Luc, F-06700 Saint-Laurent-du-Var (FR); BROUSSAUD, Olivier, F-06200 Nice (FR)
(74) Mandataire: Ores, Irène
(86) Numéro de dépôt international: FR9600635
(87) Numéro de publication internationale: WO9633743

(56) Documents cités:
- EP-A- 0 319 856
- EP-A- 0 415 567

## Description

La présente invention est relative à des compositions pulvérulentes et solubles dans l'eau, destinées à permettre la dissolution complète et rapide de principes actifs qui, habituellement, ne se solubilisent dans l'eau qu'en présence de sels basiques, tels que les antibiotiques, de la famille des béta-lactamides comme les pénicillines ou leurs dérivés, les céphalosporines ou leurs dérivés, ou d'autres principes actifs tels que les sulfamides et les quinolones, et ce, en vue de leur absorption par voie orale. De telles compositions permettent, à la fois, de solubiliser très rapidement le principe actif, quelle que soit la nature de l'eau utilisée et de fournir une solution aqueuse de principe actif stable pendant un à plusieurs jours, ce qui permet leur conservation, donc leur utilisation pendant un ou plusieurs jours.

Les principes actifs précités sont connus pour conduire rapidement, après absorption orale, à des concentrations sanguines élevées. Etant donné leur large spectre d'activité sur les bactéries à Gram positif, mais aussi sur les bactéries à Gram négatif, leurs indications thérapeutiques sont nombreuses en pharmacie humaine ou dans le domaine vétérinaire : veaux, porcs, volailles et autres.

Des dérivés solubles des pénicillines existent et ont été très largement décrits dans la littérature, tels que les sels sodique ou potassique de la pénicilline G ou le sel sodique de l'amoxicilline ou encore le sel sodique de la céfalexine. Ces composés, tout en étant très solubles, ont l'inconvénient majeur de présenter à la fois :
- une instabilité en solution, due au pH alcalin du milieu qu'ils génèrent, et
- également une instabilité pendant leur conservation à l'état de poudre.

En effet, ils sont généralement obtenus par salification en solution aqueuse, souvent à chaud, puis évaporation de l'eau par une technique classique comme la lyophilisation ou le séchage par pulvérisation ; ces différentes opérations déclenchent des réactions de dégradation, bien connues, de ces molécules, en milieu alcalin, et ce dès leur préparation, lesdites réactions de dégradation se poursuivant, même lorsque lesdits composés sont à l'état pulvérulent.

Pour obtenir des solutions aqueuses, administrables par voie orale, les sels solubles de pénicillines sont, par exemple, utilisés directement ou créés *in situ* par l'emploi de sels inorganiques (solubilisant) de sodium, potassium, magnésium, ammonium ou d'ammonium substitué, pharmaceutiquement acceptables. Dans ces deux cas, si la solubilité est acceptable, une rapide destruction du principe actif est observée, en raison du caractère basique de la solution. Cette dégradation est plus importante avec les mélanges d'antibiotiques et de sels inorganiques (solubilisant), car pour avoir la solubilité souhaitée, il faut avoir un excès de solubilisant ; or, d'une part, le sel inorganique a un caractère très basique et, d'autre part, le rapport stoechiométrique 1:1, nécessaire pour obtenir la dissolution, doit être modifié pour obtenir la meilleure solubilité possible, ce qui entraîne l'obtention d'un pH particulièrement élevé. Les mélanges d'antibiotiques et de sels inorganiques sont néanmoins plus stables à l'état pulvérulent que ne le sont les sels solubles de pénicillines.

La présente invention s'est par conséquent fixé pour but de pouvoir disposer d'une composition pulvérulente permettant d'avoir à la fois :
- une solubilité totale et rapide dans l'eau, quelles que soient les caractéristiques de cette dernière,
- une stabilité dans l'eau d'au moins 24 heures, et
- une stabilité à l'état pulvérulent d'au moins 2 ans.

La présente invention a pour objet des compositions pulvérulentes et solubles dans l'eau, caractérisées en ce qu'elles comprennent en association, sous forme pulvérulente :
. au moins un principe actif non salifié, soluble dans l'eau, uniquement à pH alcalin,
. au moins un sel de base forte, en excès et
. au moins un tampon, formé d'une ou plusieurs substances, apte à maintenir le pH du milieu, à une valeur inférieure au pH normal de solubilisation dudit principe actif.

De façon inattendue, de telles compositions pulvérulentes comprenant, en association, un sel de base forte, engagé en excès, et un tampon, c'est-à-dire une substance ou plus précisément un mélange de substances qui maintient le pH du milieu pendant et après dissolution dans un domaine de pH précis, permettent de solubiliser très rapidement le principe actif, tout en lui conservant, en solution, son intégrité pendant un à plusieurs jours. Les quantités de sel de base forte et de tampon sont fonction de la quantité et de la nature de la molécule active à solubiliser.

Selon un mode de réalisation avantageux de ladite composition pulvérulente, le sel de base forte est avantageusement choisi dans le groupe constitué par les sels basiques comprenant en tant qu'anion, des ions carbonate, bicarbonate, sulfate, et en tant que cation, des ions sodium, potassium, ammonium et ammonium substitué. Le carbonate de sodium sera choisi préférentiellement.

Selon un autre mode de réalisation avantageux de ladite composition pulvérulente, le tampon est avantageusement choisi dans le groupe qui comprend les tampons phosphates conduisant à des pH compris entre 6,5 et 8,0, les tampons chlorure de sodium/phosphate disodique conduisant à des pH compris entre 6,8 et 7,5, les tampons chlorure de sodium/trisaminométhane voisins de pH 7,5, les tampons citro-phosphate conduisant à des pH compris entre 6,5 et 7,9, les tampons borate voisins de pH 7,5 et toutes les combinaisons possibles pour l'obtention d'un tampon au pH souhaité. Les tampons phosphates seront choisis préférentiellement.

Selon un autre mode de réalisation avantageux de ladite composition pulvérulente, elle comprend en outre au moins l'un des composés suivants : agents chélatants, agents anti-mottants (agents fixateurs d'eau), arômes ou parfums ou charges, à l'état pulvérulent.

Selon encore un autre mode de réalisation de ladite composition pulvérulente, elle contient entre 1 et 60 % (en poids par rapport à la composition pulvérulente totale) de principe actif.

La composition est réalisée et conditionnée, de manière connue de l'Homme du métier, par mélange à sec des composants en poudre et emballage dans des conditionnements adaptés à ce type de formulations.

De telles compositions pulvérulentes, solubles dans l'eau, trouvent notamment application dans les traitements par voie orale, en particulier en élevage industriel. Leur solubilité et leur stabilité en solution permettent, par exemple dans le domaine vétérinaire, de dispenser le médicament d'une façon homogène, à travers les installations de distribution d'eau de boisson, notamment les installations automatiques des élevages industriels. L'eau de boisson est un très bon vecteur pour faire absorber, de manière curative ou préventive, les médicaments aux animaux. On peut suivre parfaitement un schéma thérapeutique, le moduler suivant la nécessité et le poursuivre sur plusieurs jours, si besoin.

La posologie, par exemple pour l'amoxicilline, est de 10 mg de principe actif par kg de poids vif et par jour (mg.kg⁻¹.j⁻¹). Cela conduit à avoir une concentration en principe actif, dans l'eau de boisson, selon le poids de l'animal et sa consommation journalière d'eau, comprise entre 50 et 200 mg par litre. Le traitement doit être fait sur 3 à 5 jours. Cette posologie peut être doublée le 1er jour dans les cas graves soit entre 100 et 400 mg par litre d'eau.

La présente invention a également pour objet des compositions sous forme de solutions aqueuses, caractérisées en ce qu'elles comprennent une composition pulvérulente selon l'invention et de l'eau.

Selon la quantité d'eau associée à ladite composition pulvérulente, on obtient des solutions concentrées ou des solutions diluées de principe actif ; pour les solutions très concentrées, la stabilité peut n'être que de quelques heures et, pour les solutions diluées, qui contiennent les doses efficaces de principes actifs, la stabilité doit être d'au moins de 24 heures.

Le fait que même les solutions concentrées présentent une stabilité d'au moins quelques heures, facilite la préparation et l'emploi des solutions diluées, qui sont effectivement dispensées.

Comme précisé ci-dessus, la solution médicamenteuse peut être obtenue directement, en dissolvant la quantité nécessaire de principe actif dans le volume d'eau requis. Cette façon d'opérer est surtout utilisée pour traiter peu d'animaux, c'est-à-dire quand le volume d'eau n'est pas trop important sinon cela serait de manipulation peu aisée. Cette dissolution directe est aussi utilisée pour des gros volumes dans des cuves munies d'une agitation. Ceci montre l'importance cruciale d'avoir une très bonne et rapide dissolution.

Pour la préparation de plus gros volumes qui sont distribués à partir de cuves, il est plus facile de préparer une solution concentrée dans un récipient, tel qu'un seau, c'est-à-dire que la quantité de principe actif nécessaire est dispersée dans un volume d'eau de 20 à 100 fois plus petit que le volume final. La solution concentrée est ensuite transférée dans la cuve contenant le solde nécessaire d'eau. Une bonne dissolution, même quand la solution est concentrée, permet de verser dans la cuve un principe actif en solution et non en suspension, ce qui évite les problèmes liés à la manipulation d'une suspension, à savoir une sédimentation en fond de cuve, entraînant de nombreux problèmes techniques comme l'obturation de la tuyauterie de distribution.

De manière inattendue, les solutions selon l'invention, préparées à partir des compositions pulvérulentes telles que définies ci-dessus, permettent effectivement d'obtenir :
- une solution vraie, importante pour l'homogénéité ;
- une stabilité en solution, de telle sorte que les recristallisations sont évitées ;
- une non-dégradation du principe actif en solution, de telle sorte que le traitement puisse être préconisé sur un jour ou plus.

La présente invention a également pour objet un procédé de préparation desdites compositions sous forme de solutions aqueuses, caractérisé en ce qu'il comprend :
(i) la préparation d'une composition pulvérulente selon l'invention et
(ii) la solubilisation de ladite composition dans une quantité d'eau suffisante pour l'obtention dans la solution finale d'une quantité de principe actif par kg d'animal à traiter efficace pour au moins 24 heures.

De manière avantageuse, comme précisé ci-dessus, l'étape (ii) peut être réalisée en deux stades, à savoir préparation d'une solution concentrée et dilution de la solution concentrée, de manière à obtenir dans la solution finale une quantité de principe actif par kg d'animal à traiter efficace pour au moins 24 heures.

De telles compositions sous forme de solutions aqueuses sont stables pendant 1 à plusieurs jours.

Outre les dispositions qui précèdent, l'invention comprend encore d'autres dispositions, qui ressortiront de la description qui va suivre, qui se réfère à des exemples d'association objet de la présente invention.

Il doit être bien entendu, toutefois, que ces exemples sont donnés à titre d'illustration de l'objet de l'invention, dont ils ne constituent en aucune manière une limitation.

### EXEMPLE 1 : Composition soluble contenant 10 pour cent d'amoxicilline (Composition A).

### 1) Préparation d'une composition pulvérulente conforme à l'invention :

On mélange mécaniquement de l'amoxicilline (sous forme trihydrate) (10 g, soit 27,4 mmoles) avec du carbonate de sodium anhydre (3 g, soit 28,3 mmoles), du phosphate monosodique dihydrate (13 g), du phosphate disodique (1 g) et du dextrose anhydre, charge soluble qui permet d'ajuster les concentrations, de manière à avoir une composition à 10 % en principe actif (p/p) (71,2 g) (= composition A).

### 2) Préparation de la solution aqueuse :

40 g de la composition A sont dissous dans 1000 ml d'eau du robinet.

### 3) Etude comparative des propriétés des compositions selon l'invention avec des compositions de l'Art antérieur :

### a) test de solubilité.

Les vitesses de dissolution de la composition A décrite ci-dessus et de l'amoxicilline sodique ont été comparées :

Les mêmes quantités d'amoxicilline soit :
(1) 40 g de la composition A, équivalents à 4 g d'amoxicilline (sous forme trihydrate) et
(2) 4 g d'amoxicilline (sous forme sodique), ont été dissoutes dans 1000 ml d'eau du robinet ; le temps de dissolution et le pH de chacune des solutions obtenues ont été notés.

| Produit | Temps de dissolution | pH final |
|---|---|---|
| (1) Composition A (40 g) | 45 sec | 7,7 |
| (2) Amoxicilline (Na) (4 g) | 42 sec | 8,7 |

La solubilité de la composition A est comparable à celle du sel sodique d'amoxicilline, forme la plus soluble de cet antibiotique. Il est à noter que, si les vitesses de dissolution sont équivalentes, les dissolutions ont été obtenues dans des domaines différents de pH.

### b) rôle de chaque constituant de la composition : étude de l'aspect de la solution ou suspension obtenue, de sa solubilité, de son pH.

Les mêmes quantités d'amoxicilline (sous forme trihydrate), soit 4 g ont été ajoutées à des solutions constituées pour :
- la première : de carbonate de sodium (3 g) et d'eau (1000 ml);
- la deuxième : de phosphate monosodique dihydrate (13 g), de phosphate disodique (1 g) et d'eau (1000 ml), et
- la troisième : de carbonate de sodium (3 g), de phosphate monosodique dihydrate (13 g), de phosphate disodique (1 g) et d'eau (1000 ml), équivalente à l'association sel de base forte-tampon, utilisée dans la composition A. L'aspect, le temps de dissolution et le pH de chacune des solutions obtenues ont été notés.

| Solvant | Aspect de la solution | Temps de dissolution | pH final |
|---|---|---|---|
| 1er solvant carbonate de Na | solution | 45 sec | 8,5 |
| 2ème solvant tampon | présence d'insolubles | non déterminé | 6,5 |
| 3ème solvant carbonate de Na et tampon | solution | 45 sec | 7,6 |

### c) étude comparative de la stabilité de l'amoxicilline (sous forme trihydrate) engagée dans la composition A, associée seulement avec un sel de base forte, et de l'amoxicilline sodique.

### c-1 essai "solution normale d'emploi" :

Les mêmes quantités d'amoxicilline, soit :
(1) 0,8 g de la composition A (soit 80 mg d'amoxicilline (sous forme trihydrate)),
(2) 80 mg d'amoxicilline (sous forme trihydrate) associés à 24 mg de carbonate de sodium, et
(3) 80 mg d'amoxicilline (sous forme sodique) ont été dissoutes dans 1000 ml d'eau du robinet (dose efficace requise pour traiter les volailles).

| Produit | pH | Teneur en amoxicilline en solution au temps (en heures) | | | | |
|---|---|---|---|---|---|---|
| | | t₀ | t₄ | t₁₂ | t₂₄ | t₃₆ |
| (1) Composition A (0,8 g) | 7,6 | 100,5 | 99,2 | 100,8 | 101,0 | 98,7 |
| (2) Amoxicilline (3H₂O) (80 mg) carbonate de Na (24 mg) | 9,2 | 100,1 | 87,8 | 66,2 | - | - |
| (3) Amoxicilline (Na) (80 mg) | 8,7 | 100,0 | 92,1 | 75,4 | - | - |

La composition A, objet de la présente invention, conduit en solution à une stabilité de l'amoxicilline, sur une période de plus de 36 heures comparativement à l'amoxicilline sodique d'une part et à l'amoxicilline trihydrate associée à un sel inorganique de sodium (équivalent au sel utilisé dans la composition A) d'autre part. De manière inattendue, la solubilisation à un pH non alcalin permet effectivement une amélioration significative de la stabilité ; en conséquence, les valeurs de pH sont une des clés des instabilités ou stabilités des compositions aqueuses obtenues.

### c-2 essai << solution concentrée >> :

Les mêmes quantités d'amoxicilline soit :
(1) 40 g de la composition A (soit 4 g d'amoxicilline (sous forme trihydrate)),
(2) 4 g d'amoxicilline (sous forme trihydrate) associés à 1,2 g de carbonate de sodium, et
(3) 4 g d'amoxicilline (sous forme sodique), ont été dissoutes séparément dans 1000 ml d'eau du robinet (concentration utilisée pour réaliser une pré-solubilisation avant dilution pour utilisation).

| Produit | pH | Teneur en amoxicilline en solution en % au temps (en heures) | | | |
|---|---|---|---|---|---|
| | | t₀ | t₄ | t₁₂ | t₂₄ |
| (1) Composition A (40 g) | 7,7 | 100,2 | 99,2 | 98,3 | 97,1 |
| (2) Amoxicilline (3H₂O) (4 g) carbonate de Na (1,2 g) | 9,4 | 100,2 | 80,3 | - | - |
| (3) Amoxicilline (Na) (4 g) | 8,7 | 100,0 | 87,2 | - | - |

De même qu'au paragraphe précédent (c-1), une meilleure stabilité de l'amoxicilline dans une composition selon l'invention, en solution est relevée, comparativement à l'amoxicilline sodique et à l'amoxicilline trihydrate associée au carbonate de sodium. Compte-tenu de la forte teneur en principe actif des solutions concentrées, une stabilité moins longue, 12 heures au lieu de 36 heures pour la solution à base de composition A selon le paragraphe c-1, est enregistrée. Ce temps est largement suffisant pour la préparation des solutions utiles thérapeutiquement et stables plus de 24 heures en solution.

### d) Stabilité dans le temps

Les stabilités, à la température ambiante (25°C) de la composition A, de l'amoxicilline trihydrate, d'une composition A', équivalente à la composition A sans le tampon, soit 10 g d'amoxicilline (sous forme trihydrate) avec 3 g de carbonate de sodium anhydre et du dextrose anhydre (85,2 g), et de l'amoxicilline sodique, conditionnées dans des sachets, habituellement utilisés pour ce genre de spécialité, ont été étudiées pendant 24 mois.

| Produit | Titre de l'amoxicilline en % au temps (en mois) | | | |
|---|---|---|---|---|
| | t₀ | t₆ | t₁₂ | t₂₄ |
| Composition A | 100,5 | 99,6 | 100,2 | 98,9 |
| Amoxicilline (3H₂O) | 100,0 | 99,2 | 99,6 | 98,5 |
| Composition A' | 99,8 | 98,8 | 97,8 | 93,7 |
| Amoxicilline Na | 100,0 | 97,3 | 94,8 | 90,2 |

La stabilité de l'amoxicilline à l'état pulvérulent, engagée dans la composition A est comparable à celle de la matière active seule, l'amoxicilline trihydrate qui est la référence.

Par contre, l'amoxicilline trihydrate, associée seulement au solubilisant, le carbonate de sodium, montre une instabilité au delà de 12 mois, instabilité qui apparaît à 6 mois pour l'amoxicilline sodique, forme salifiée soluble.

### EXEMPLE 2 : Composition soluble contenant 50 % d'amoxicilline.

De façon similaire à l'exemple 1, on mélange mécaniquement de l'amoxicilline (sous forme trihydrate) (50 g, soit 137 mmoles) avec du carbonate de sodium anhydre (15 g, soit 141,5 mmoles), du phosphate monosodique dihydrate (5 g), de l'hexamétaphosphate sodique (20 g) et du dextrose anhydre (10 g).

Les mêmes quantités d'amoxicilline, soit 10 g de la composition décrite ci-dessus, équivalents à 5 g d'amoxicilline (sous forme trihydrate), et 5 g d'amoxicilline (sous forme sodique) ont été dissoutes séparément dans 1000 ml d'eau du robinet de dureté 36° ; le temps de dissolution et le pH de chacune des solutions obtenues ont été notés.

| Produit | Temps de dissolution | pH final |
|---|---|---|
| Composition (10 g) | 46 sec | 7,75 |
| Amoxicilline (Na) (5 g) | 1 min 15 sec reste des insolubles | 8,8 |

La dureté de l'eau freine la solubilisation du principe actif en entraînant la formation de sel calcique de l'antibiotique insoluble. La composition, réalisée selon la présente invention, permet de solubiliser l'antibiotique en faisant abstraction de la qualité de l'eau utilisée.

### EXEMPLE 3: Composition soluble contenant 20 % de sulfaméthazine.

De façon similaire à l'exemple 1, on mélange mécaniquement du sulfaméthazine (20 g, soit 71,85 mmoles) avec du carbonate de sodium anhydre (8 g, soit 75,47 mmoles), du chlorure de sodium (18 g), du trisaminométhane (5 g), un anti-mottant (1 g) et du dextrose anhydre (48 g).

Ce sulfamide est donné pour lutter contre les affections à germes Gram positif (*Staphylococcus, Listeria*...), à germes Gram négatif (*E. coli, Klebsiella*, *Enterobacter, Proteus, Salmonella*...), et contre les coccidies chez les veaux, agneaux, chevreaux, porcins, lapins et volailles. La posologie est de 1,3 g à 1,7 g de sulfaméthazine, soit 6,5 à 8,5 g de composition/litre d'eau de boisson pour les lapins et les volailles.

### EXEMPLE 4: Composition soluble pédiatrique contenant 30 % de céfalexine (sous forme monohydrate).

De façon similaire à l'exemple 1, on mélange mécaniquement de la céfalexine (sous forme monohydrate) (30 g, soit 86,4 mmoles) avec du carbonate de sodium anhydre (11 g, soit 103,77 mmoles), de l'acide citrique (7 g), de l'aspartam (0,5 g), un anti-mottant (1 g), une composition aromatique (5 g), et du phosphate disodique (41,5 g).

La composition est répartie dans des flacons de verre gradués à 120 ml à raison de 10 g par flacon, soit 3 g de céfalexine (sous forme monohydrate). La solution homogène de céfalexine est reconstituée par l'ajout d'eau en quantité suffisante pour obtenir 120 ml. Cette solution peut se conserver plusieurs jours. Elle est donnée aux enfants à raison de 1 à 2 ml/par kg/jour, de préférence en 2 ou 3 prises au cours de la journée.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Compositions pulvérulentes et solubles dans l'eau, **caractérisées en ce qu'**elles comprennent en association, sous forme pulvérulente :
. au moins un principe actif non salifié, soluble dans l'eau, uniquement à pH alcalin,
. au moins un sel de base forte, en excès et
. au moins un tampon, formé d'une ou plusieurs substances, apte à maintenir le pH du milieu, à une valeur inférieure au pH normal de solubilisation dudit principe actif.

2. Composition pulvérulente selon la revendication 1, **caractérisée en ce que** le sel de base forte est avantageusement choisi dans le groupe constitué par les sels basiques comprenant en tant qu'anion, des ions carbonate, bicarbonate, sulfate, et en tant que cation, des ions sodium, potassium, ammonium et ammonium substitués.

3. Composition pulvérulente selon la revendication 1 ou la revendication 2, **caractérisée en ce que** le tampon est avantageusement choisi dans le groupe qui comprend les tampons phosphates conduisant à des pH compris entre 6,5 et 8,0, les tampons chlorure de sodium/phosphate disodique conduisant à des pH compris entre 6,8 et 7,5, les tampons chlorure de sodium/trisaminométhane voisins de pH 7,5, les tampons citro-phosphate conduisant à des pH compris entre 6,5 et 7,9, les tampons borate voisins de pH 7,5.

4. Composition pulvérulente selon l'une quelconque des revendications 1 à 3, **caractérisée en ce qu'**elle comprend en outre au moins l'un des composés suivants : agents chélatants, agents anti-mottants, arômes ou parfums ou charges, à l'état pulvérulent.

5. Composition pulvérulente selon l'une quelconque des revendications 1 à 4, **caractérisée en ce qu'**elle contient entre 1 et 60 % (en poids par rapport à la composition pulvérulente totale) de principe actif.

6. Compositions sous forme de solutions aqueuses, **caractérisées en ce qu'**elles comprennent une composition pulvérulente selon l'une quelconque des revendications 1 à 5 et de l'eau.

7. Procédé de préparation desdites compositions sous forme de solutions aqueuses, **caractérisé en ce qu'**il comprend :
(i) la préparation d'une composition pulvérulente selon l'une quelconque des revendications 1 à 5 et
(ii) la solubilisation de ladite composition dans une quantité d'eau suffisante pour l'obtention dans la solution finale d'une quantité de principe actif par kg d'animal à traiter efficace pour au moins 24 heures.

8. Procédé de préparation selon la revendication 7, **caractérisé en ce que** l'étape (ii) peut être réalisée en deux stades, à savoir préparation d'une solution concentrée et dilution de la solution concentrée, de manière à obtenir dans la solution finale une quantité de principe actif par kg d'animal à traiter efficace pour au moins 24 heures.

9. Composition pharmaceutique à administration orale à usage vétérinaire, **caractérisée en ce qu'**elle comprend une composition selon l'une quelconque des revendications 1 à 6, éventuellement associée à au moins un véhicule pharmaceutiquement acceptable.

10. Composition pharmaceutique à administration orale à usage humain, **caractérisée en ce qu'**elle comprend une composition selon l'une quelconque des revendications 1 à 6, éventuellement associée à au moins un véhicule pharmaceutiquement acceptable.

## Claims

1. Pulverulent, water-soluble compositions, **characterized in that** they comprise, in combination and in pulverulent form:
· at least one non-salified active ingredient which is only soluble in water at alkaline pH,
· at least one salt of a strong base, which is present in excess, and
· at least one buffer, which is formed from one or more substances which are suitable for maintaining the pH of the medium at a value which is lower than the normal pH for solubilizing the said active ingredient.

2. Pulverulent composition according to Claim 1, characerized in that the salt of the strong base is advantageously selected from the group consisting of basic salts which comprise, as anion, carbonate, bicarbonate or sulphate ions and, as cation, sodium, potassium, ammonium or substituted ammonium ions.

3. Pulverulent composition according to Claim 1 or Claim 2, **characterized in that** the buffer is advantageously selected from the group which comprises phosphate buffers, leading to pH values of between 6.5 and 8.0, sodium chloride/disodium phosphate buffers, leading to pH values of between 6.8 and 7.5, sodium chloride/trisaminomethane buffers in the neighbourhood of pH 7.5, citro-phosphate buffers leading to pH values of between 6.5 and 7.9, and borate buffers in the neighbourhood of pH 7.5.

4. Pulverulent composition according to any one of Claims 1 to 3, **characterized in that** it additionally comprises at least one of the following compounds: chelating agents, anti-caking agents, flavours or perfumes or fillers, in the pulverulent state.

5. Pulverulent composition according to any one of Claims 1 to 4, **characterized in that** it contains between 1 and 60% (by weight based on the total pulverulent composition) of active ingredient.

6. Compositions in the form of aqueous solutions, **characterized in that** they comprise a pulverulent composition according to any one of Claims 1 to 5 and water.

7. Process for preparing the said compositions in the form of aqueous solutions, **characterized in that** it comprises:
(i) preparing a pulverulent composition according to any one of Claims 1 to 5, and
(ii) solubilizing the said composition in a quantity of water which is sufficient for obtaining, in the final solution, a quantity of active ingredient per kg of animal to be treated which is effective for at least 24 hours.

8. Preparation process according to Claim 7, **characterized in that** step (ii) can be carried out in two stages, namely preparation of a concentrated solution and dilution of the concentrated solution so as to obtain, in the final solution, a quantity of active ingredient per kg of animal to be treated which is effective for at least 24 hours.

9. Pharmaceutical composition for veterinary use to be administered orally, **characterized in that** it comprises a composition according to any one of Claims 1 to 6, possibly combined with at least one pharmaceutically acceptable excipient.

10. Pharmaceutical composition for human use to be administered orally, **characterized in that** it comprises a composition according to any one of Claims 1 to 6, possibly combined with at least one pharmaceutically acceptable excipient.

## Patentansprüche

1. Feinpulverige und wasserlösliche Zusammensetzungen, **dadurch gekennzeichnet, dass** sie in Zuordnung und in feinpulveriger Form umfassen:
- wenigstens einen ausschließlich bei alkalischem pH aktives, nichtsalzbildendes wasserlöslichen Stoff,
- wenigstens ein Salz einer starken Base im Überschuss und
- wenigstens einen Puffer, der aus einer oder mehreren Substanzen gebildet ist, ausgelegt um den pH des Milieus auf einen Wert unter dem normalen Löslichkeits-pH dieses aktiven Stoffes zu erhalten.

2. Feinpulverige Zusammensetzung gemäß Anspruch 1, **dadurch gekennzeichnet, dass** das Salz einer starken Base vorteilhaft in der durch die basischen Salze gebildeten Gruppe gewählt ist, die als Anion Carbonat-, Bicarbonat-, Sulfationen und als Kation Natrium-, Kalium-, Ammonium und substituierte Ammoniumionen umfassen.

3. Feinpulverige Zusammensetzung nach Anspruch 1 oder 2, **dadurch gekennzeichnet, dass** der Puffer vorteilhaft aus der Gruppe gewählt wird, die die Phosphatpuffer, welche zu pH-Werten zwischen 6,5 und 8,0 führen, die Natriumchlorid/Dinatriumphosphatpuffer, welche zu pH-Werten zwischen 6,8 und 7,5 führen, die Natriumcholrid/Trisaminomethanpuffer, nahe pH 7,5, die Citro-Phosphatpuffer, welche zu pH-Werten zwischen 6,5 und 7,9 führen, die Boartpuffer nahe pH 7,5 umfasst.

4. Feinpulverige Zusammensatzung nach einem der Ansprüche 1 bis 3, **dadurch gekennzeichnet, dass** sie im übrigen wenigstens eine der folgenden Verbindungen umfasst: chelatbildende Reagenzien, Antiverklumpungsreagenzien, Aromen oder Parfüme oder Füllstoffe in feinpulverigem Zustand.

5. Feinpulverige Zusammensetzung nach einem der Ansprüche 1 bis 4, **dadurch gekennzeichnet, dass** sie zwischen 1 und 60 % (in Gewicht im Verhältnis zur gesamten feinpulverigen Zusammensetzung) an aktivem Stoff enthält.

6. Zusammensetzungen in Form von wässerigen Lösungen, **dadurch gekennzeichnet, dass** diese eine feinpulverige Zusammensetzung nach einem der Ansprüche 1 bis 5 und Wasser umfassen.

7. Verfahren zur Herstellung dieser Zusammensetzungen in Form von wässerigen Lösungen, **dadurch gekennzeichnet, dass** es umfasst:
(i) die Herstellung einer feinpulverigen Zusammensetzung nach einem der Ansprüche 1 bis 5 und
(ii) das Solubilisieren dieser Zusammensetzung in einer zum Erhalt ausreichenden Menge in der Endlösung einer Menge von aktivem Stoff pro kg zu behandelndem Tier, die für wenigstens 24 Stunden wirksam ist.

8. Verfahren zur Herstellung nach Anspruch 7, **dadurch gekennzeichnet, dass** die Stufe (ii) in zwei Stadien durchgeführt werden kann, nämlich Herstellung einer konzentrierten Lösung und Verdünnen der konzentrierten Lösung derart, dass in der Endlösung eine Menge von aktivem Stoff pro kg zu behandelndem Tier erhalten wird, die für wenigstens 24 Stunden wirksam ist.

9. Pharmazeutische Zusammensetzung zur oralen Verabreichung bei veterinärer Anwendung, **dadurch gekennzeichnet, dass** sie eine Zusammensetzung nach einem der Ansprüche 1 bis 6 umfasst, die gegebenenfalls wenigstens einem pharmazeutisch verträglichen Vehikel zugeordnet ist.

10. Pharmazeutische Zusammensetzung zur oralen Verabreichung bei humaner Anwendung, **dadurch gekennzeichnet, dass** sie eine Zusammensetzung nach einem der Ansprüche 1 bis 6 umfasst, die gegebenenfalls einem pharmazeutisch verträglichen Vehikel zugeordnet ist.
